(19) Europäisches Patentamt
European Patent Office
Office européen des brevets

(11) Publication number: 0 330 470
A2

# (12) EUROPEAN PATENT APPLICATION

(21) Application number: 89301774.9

(22) Date of filing: 23.02.89

(51) Int. Cl.4: C 07 D 211/90
C 07 D 405/06, A 61 K 31/44

(30) Priority: 24.02.88 JP 41369/88

(43) Date of publication of application:
30.08.89 Bulletin 89/35

(84) Designated Contracting States: DE FR GB

(71) Applicant: AJINOMOTO CO., INC.
5-8, Kyobashi 1-chome, Chuo-ku
Tokyo 104 (JP)

JAPANESE FOUNDATION FOR CANCER RESEARCH
37-1 Kamiikebukuro 1-chome Toshima-ku
Tokyo (JP)

(72) Inventor: Toyota, Kouzou
No.1-1 Suzuki-cho Kawasaki-ku
Kawasaki-shi Kanagawa-ken (JP)

Shinkai, Hisashi
c/o Ajinomoto Co., Inc. No. 5-8 Kyobashi 1 chome
Chuo-ku Tokyo (JP)

Etou, Hirozumi c/o Central Research Laboratories
Ajinomoto Co.Inc. no.1-1 Suzuki-cho Kawasaki-ku
Kawasaki-shi (JP)

Kamimura, Akira c/o Cental Research Laboratories
Ajinomoto Co.Inc. no.1-1 Suzuki-cho Kawasaki-ku
Kawasaki-shi (JP)

Eguchi, Chikahiko c/o Cental Research Laboratorie
Ajinomoto Co.Inc. no.1-1 Suzuki-cho Kawasaki-ku
Kawasaki-shi (JP)

Oosumi, Kouji c/o Cental Research Laboratories
Ajinomoto Co.Inc. no.1-1 Suzuki-cho Kawasaki-ku
Kawasaki-shi (JP)

Turuo, Takashi
No. 5-42-4-303 Yoyogi
Shibuya -ku Tokyo (JP)

(74) Representative: Armitage, Ian Michael et al
MEWBURN ELLIS & CO. 2/3 Cursitor Street
London EC4A 1BQ (GB)

## (54) 1,4-Dihydropyridine derivatives useful against tumour cells.

(57) This invention provides 1,4-dihydropyridine derivatives based on the general formula

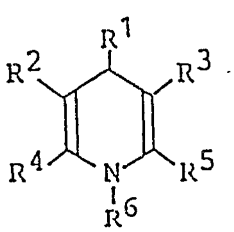

[I]

such as dimethyl 1,4-dihydro-4-(3,4-dimethoxyphenyl)-2,6-dimethyl-1-(3-phenylpropyl)-3,5-pyridinedicarboxylate.

These derivatives are active against drug-resistant tumour cells by acting to inhibit the excretion of chemotherapeutic agents from the tumour cells.

EP 0 330 470 A2

## Description

### 1,4-DIHYDROPYRIDINE DERIVATIVES USEFUL AGAINST TUMOUR CELLS

The present invention relates to 1,4-dihydropyridine derivatives (some being novel compounds) and their use against tumour cells which have acquired resistance to one or more drugs being used as chemotherapeutic agents.

Where chemotherapy is used to treat cancers, the anti-cancer agent being administered, frequently becomes ineffective during treatment, a phenomenon known as 'acquired drug resistance'. In some cases this resistance can be to several kinds of anti-cancer agents i.e. a 'multidrug resistance'.

It has been shown that the tumour cells which have acquired this multidrug resistance show enhanced excretion of the anti-cancer agents which are being administered as a treatment for the cancer. (T. Tsuruo, Pharmacia Review, No. 23 , 115-125 (1987)).

It is not practicable to overcome this problem by simply administering larger doses of the anti-cancer agent(s) as these agents have severe side-effects. Animal tests have suggested that the problem may be overcome by administering the anti-cancer agent(s) in combination with a calcium antagonist, (T. Tsuruo, Pharmacia Review, No. 23, 115-125 (1987)). However, the use of calcium antagonists has not provided a practicable solution to the problem, as the amounts of calcium needed in the above context are highly toxic and cause a lowering of blood pressure. Thus, there is a need for therapeutic agents which may help overcome the problems of acquired drug resistance.

The present invention provides 1,4-dihydropyridine derivatives represented by the following formula (I) and salts thereof. These compounds act against drug-resistant tumour cells.

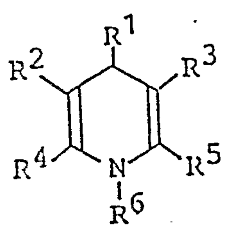

(I)

In the above formula (I):

$R^1$ is aryl (suitably $C_{6-14}$ e.g., phenyl or naphthyl) which may optionally have one or more substituent group(s) (suitably $C_{1-5}$);

$R^2$ and $R^3$ are the same or different organic groups selected from alkyl (e.g., $C_{1-5}$) such as methyl, ethyl, isopropyl alkyloxycarbonyl (suitably with $C_{1-5}$ alkyl segment such as methyl, ethyl, tert-butyl, isopropyl) aryloxycarbonyl (e.g., phenyloxycarbonyl and naphthyloxycarbonyl), aralkyloxycarbonyl (e.g., benzyloxycarbonyl), aminocarbonyl, hydroxycarbonyl, formyl, hydroxyalkyl (e.g. hydroxymethyl), alkylaminocarbonyl (e.g. methylaminocarbonyl ), arylaminocarbonyl (e.g. benzylaminocarbonyl) and cyano, all of which may optionally have one or more substituent groups;

$R^4$ and $R^5$ are the same or different groups selected from hydrogen, lower alkyl (suitably $C_{1-5}$ e.g., methyl and ethyl), hydroxy-methyl, cyano, amino, formyl groups, or halo (e.g. fluorine, chlorine, bromine and iodine); and

$R^6$ is alkyl (suitably $C_{1-10}$), aralkyl (suitably with $C_{1-10}$ alkyl moiety, e.g., phenylalkyl), alkoxycarbonyl (suitably with $C_{1-5}$ alkyl segment), or aralkyloxycarbonyl (suitably with $C_{1-5}$ alkyl moiety e.g., a phenylalkyloxycarbonyl), all of which may optionally have one or more substituent groups and the alkyl segment in each may optionally contain carbon-carbon double or triple bonds or hetero atoms (suitably O, N or S).

For $R^1$, examples of the aryl group substituents are halogen (e.g., fluorine, chlorine, bromine and iodine), alkyl (suitably $C_{1-5}$ e.g., methyl, ethyl, propyl and isopropyl), hydroxy, alkyloxy (suitably $C_{1-5}$ e.g., methoxy and ethoxy), benzyloxy, alkylene dioxy, (suitably $C_{1-3}$) nitro, amino, nitrile and trifluoromethyl.

$R^2$ and $R^3$ each may be methyl, hydroxymethyl, methoxycarbonyl, ethoxycarbonyl, isopropoxycarbonyl, methoxyethyloxycarbonyl, tert-butoxycarbonyl, phenoxycarbonyl, benzyloxycarbonyl, aminocarbonyl, methylaminocarbonyl, benzylaminocarbonyl, hydroxycarbonyl, formyl, or cyano.

When $R^6$ is an aralkyl group, it may optionally have substituted or unsubstituted phenyl or phenoxy groups at the end of its alkylene segment, and the alkylene segment may optionally contain one or more substituent group(s) selected from alkyl (suitably $C_{1-5}$ e.g., methyl, ethyl and isopropyl), nitrile or oxo. The alkylene segment may also contain carbon-carbon double or triple bonds (representing an alkenylene group of 3 to 9 carbon atoms, or an alkynylene group of 3 to 9 carbon atoms) or hetero atoms (e.g. O, N or S). The phenyl ring substituent, may be selected from halogen (e.g., fluorine, chlorine, bromine and iodine), alkyl (suitably $C_{1-5}$ e.g., methyl, ethyl, propyl and isopropyl), hydroxy, alkyloxy (suitably $C_{1-5}$ e.g., methoxy and ethoxy), alkylene dioxy, (suitably $C_{1-3}$ benzyloxy, nitro, amino), nitrile and trifluoromethyl.

Of the 1,4-dihydropyridine derivatives of formula (I), those which are represented by the following general

formula (II) are novel compounds with high activity from which some new physiological activities may be expected:

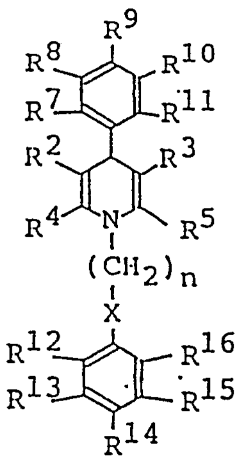

(II)

In the above formula II:
$R^2$ and $R^3$ are the same or different organic groups selected from alkyl (e.g., $C_{1-5}$ such as methyl, ethyl, isopropyl),alkyloxycarbonyl (suitably with $C_{1-5}$ alkyl segment such as methyl, ethyl, tert-butyl, isopropyl) aryloxycarbonyl (e.g., phenyloxycarbonyl and naphthyloxycarbonyl), aralkyloxycarbonyl (e.g., benzyloxycarbonyl), aminocarbonyl, hydroxycarbonyl, formyl, hydroxyalkyl (e.g. hydroxymethyl), alkylaminocarbonyl (e.g. methylaminocarbonyl), arylaminocarbonyl (e.g. benzylaminocarbonyl) and cyano, all of which may optionally have one or more substituent groups;
$R^4$ and $R^5$ are the same or different selected from hydrogen, methyl or ethyl;
$R^7$, $R^8$, $R^9$, $R^{10}$ and $R^{11}$ are the same or different selected from hydrogen, halogen (e.g., fluorine, chlorine, bromine and iodine), alkyl (suitably $C_{1-5}$ e.g., methyl, ethyl, propyl and isopropyl), hydroxy, alkyloxy (suitably $C_{1-5}$ e.g., methoxy and ethoxy, nitro, amino), nitrile and trifluoromethyl and wherein any neighbouring two of $R^7$ to $R^{11}$ may be bonded by a $C_{1-3}$ alkylenedioxy group.

$R^{12}$, $R^{13}$, $R^{14}$, $R^{15}$ and $R^{16}$ are the same or different selected from hydrogen, halogen, (e.g., fluorine, chlorine, bromine and iodine), alkyl (suitably $C_{1-5}$ e.g. methyl, ethyl, propyl and isopropyl), hydroxy, alkyloxy (suitably $C_{1-5}$ e.g., methoxy and ethoxy), alkylene dioxy, (suitably $C_{1-3}$), benzyloxy, nitro, amino, nitrile and trifluoromethyl and wherein any neighbouring two of $R^7$ to $R^{11}$ may be bonded by a $C_{1-3}$ alkylenedioxy group.
X is selected from methylene, oxygen, nitrogen or carbonyl groups. Where X is methylene, it may be optionally substituted with a group selected from alkyl, (suitably $C_{1-5}$) or nitrile.
n is an integer of 2 to 9.

In the above definitions where the group is amino or aminocarbonyl it may be optionally substituted with a $C_{1-14}$ organo group, for example methyl, dimethyl, dibenzyl.

The novel substances of this invention may also be in the form of salts. When the 1,4-dihydropyridine derivatives are acidic they may be salts of alkali metals such as sodium or potassium, salts of alkali earth metals such as calcium, salts of inorganic bases such ammonia, salts of organic bases such as cyclohexylamine, N-methyl-D-glucosamine, salts of basic amino acids such as lysine or arginine, or salts of several kinds of cations.

When the 1,4-dihydropyridine derivatives are basic they may be salts of mineral acids such as hydrochloric or sulphuric acids or of organic acids such as tartaric or malic acids or they may be salts of acidic amino acids such as glutamic or aspartic acids.

The 1,4-dihydropyridine derivatives of the present invention may be used in association (i.e. either separately or together) with chemotherapeutic anti-cancer agents such as vinca alkaloids (e.g. vincristine, vinblastin) antibiotics (e.g. adriamycine, bleomycine,) nucleic acids (e.g. 5-fluoro uracil) cisplatin, etopside. The chemotherapeutic anti-cancer agents when used in association with the 1,4-dihydropyridine derivatives of the present invention may be used in the same dose range as that used for normal chemotherapy.

The compounds of this invention can be prepared by means of a Hantsch type cyclocondensation reaction (Review: A.Sausis, G.Duburs; Heterocycles, 27, 269 (1988)). For example, reacting a compound of formula [III] and methyl acetoacetate [IV] by heating (under reflux) in the presence or absence of an organic solvent (e.g., methanol, ethanol, propanol, isopropanol, benzene, toluene, dioxane, tetrahydrofuran, dimethyl sulfoxide or dimethylformamide), to obtain a compound of formula [V]. The compound of formula [V] and 3-aminocrotonic acid methyl ester [VI] are reacted in the same conditions as above, to obtain a 1,4-dihydropyridine derivative of formula [VII]. A compound of formula [VII] and a compound of formula [VIII] are then reacted in an inert organic solvent (e.g., dioxane, tetrahydrofuran, dimethyl sulfoxide or diethylformamide) in the presence of sodium

hydride.

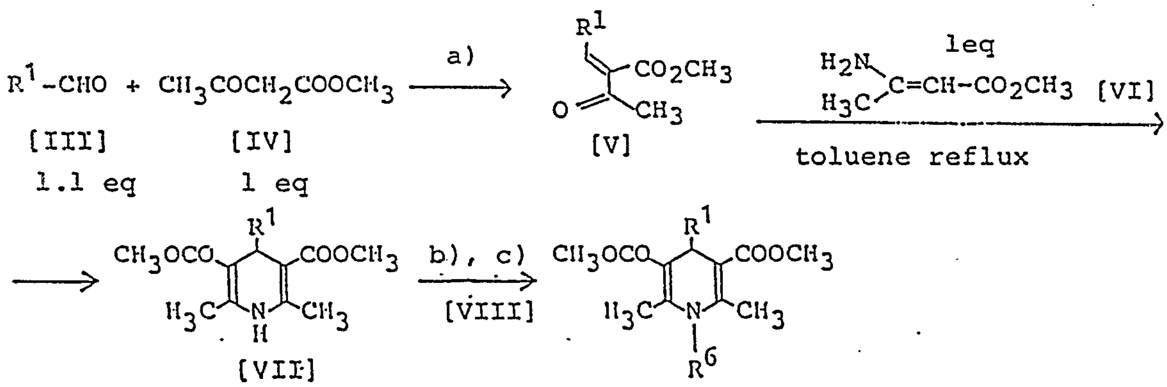


a) Piperidine and acetic acid as catalysts; benzene; reflux.

b) NaH (1.2 eq)/DMF, 70°C, 1 hour.

c) $R^6$-X (1.2 eq)/DMF, 90°C, 1 hour. (wherein $R^1$ and $R^6$ are as defined above, and X is a replaceable group such as halogen or p-toluene sulfonyloxy commonly employed in organic syntheses).

The reaction product formed is a compound of formula [I] which can be easily isolated from the reaction mixture and purified by using techniques such as solvent extraction, chromatography and crystallization well known in the art (L.F. Fieser "Experiments in Organic Chemistry").

The present invention will be further illustrated by reference to the following examples:

Example 1

Synthesis of dimethyl 1,4-dihydro-4-(3,4-dimethoxyphenyl)-2,6-dimethyl-1-(3-phenylpropyl)-3,5-pyridinedicarboxylate

a. Synthesis of methyl 2-(3,4-dimethoxybenzylidene)-acetoacetate

1.02 g (12 mmol) piperidine and 3.66 g (61 mmol) acetic acid were added dropwise and in that order, to a solution of 54.8 g (0.33 mol) 3,4-dimethoxybenzaldehyde and 34.8 mg (0.3 mol) methyl acetoacetate in 20 ml benzene. The mixture was dehydrated by heating in a Dean-Stark apparatus until the water was distilled off. The solvents were distilled off from the reaction mixture, and the oil left was allowed to stand for five days. The crystals which separated out were collected by filtration, giving 41.1 g (52%) of methyl 2-(3,4-dimethoxybenzylidene)acetoacetate as yellow crystals. M.p.: 93-95°C.

b. Synthesis of dimethyl 1,4-dihydro-4-(3,4-dimethoxyphenyl)-2,6-dimethyl-3,5-pyridinedi carboxylate

A solution of 5.0 g (18.9 mmol) 2-(3,4-dimethoxybenzylidene)acetoacetate and 2.18 g (18.9 mmol) 3-aminocrotonic acid methyl ester in 100 ml toluene was dehydrated by heating in a Dean-Stark apparatus. The solvent was distilled off from the reaction mixture, and the residue left was purified by column chromatography ($SiO_2/CHCl_3$), giving 4.30 g (63%) of dimethyl 1,4-dihydro-4-(3,4-dimethoxyphenyl)-2,6-dimethyl-3,5-pyridinedicarboxylate as whitish yellow crystals. M.p: 153.5-155°C.

c. Synthesis of dimethyl 1,4-dihydro-4-(3,4-dimethoxyphenyl)-2,6-dimethyl-1-(3-phenylpropyl)-3,5-pyridinedicarboxylate

60% sodium hydride (0.26 g) was washed with several mls of anhydrous n-hexane in an argon gas atmosphere, and 10 mls dimethylformamide was added. To the resulting mixture 20 ml of a dimethylformamide solution containing 2 g (5.5 mmol) dimethyl 1,4-dihydro-4-(3,4-dimethoxyphenyl)-2,6-dimethyl-3,5-pyridinedicarboxylate was added dropwise with stirring. The mixture was heated at 90°C for one hour. 20 ml of a dimethylformamide solution of 3-phenylpropyl bromide was then added, and heating at 90°C was continued for one hour. After cooling to room temperature, 20 ml diethyl ether was added. The resulting mixture was washed with water, and the solvent was then distilled off under reduced pressure. The crude product obtained was purified by column chromatography ($SiO_2$/n-hexane-AcOEt 2:1), producing 0.8 g (30%) of the pure product. M.p.: 97-98°C.

Examples 2 through 46

The compounds listed in the table below were prepared in the same manner as described in Example 1

| Example No. | Starting material, $R^6$-X | Product | Yield (%) | M.p. (°C) |
|---|---|---|---|---|
| 2 | Methyl iodide | Dimethyl 1,4-dihydro-4-(3,4-dimethoxyphenyl)-2,6-dimethyl-1-methyl-3,5-pyridinedicarboxylate | 48 | 155.5 -157.5 |
| 3 | Ethyl chloroformate | Dimethyl 1,4-dihydro-4-(3,4-dimethoxyphenyl)-2,6-dimethyl-1-ethoxycarbonyl-3,5-pyridinedicarboxylate | 67 | 58-60 |
| 4 | 3-Phenoxypropyl bromide | Dimethyl 1,4-dihydro-4-(3,4-dimethoxyphenyl)-2,6-dimethyl-1-(3-phenoxypropyl)-3,5-pyridinedicarboxylate | 17 | 107 -108.5 |
| 5 | 4-Phenylbutyl bromide | Dimethyl 1,4-dihydro-4-(3,4-dimethoxyphenyl)-2,6-dimethyl-1-(4-phenylbutyl)-3,5-pyridinedicarboxylate | 40 | 108 -109 |
| 6 | 5-Phenylpentyl bromide | Dimethyl 1,4-dihydro-4-(3,4-dimethoxyphenyl)-2,6-dimethyl-1-(5-phenylpentyl)-3,5-pyridinedicarboxylate | 32 | 84-84.5 |
| 7 | 4-Phenoxybutyl bromide | Dimethyl 1,4-dihydro-4-(3,4-dimethoxyphenyl)-2,6-dimethyl-1-(4-phenoxybutyl)-3,5-pyridinedicarboxylate | 20 | Oil |
| 8 | 4-(4-Methoxyphenyl)butyl bromide | Dimethyl 1,4-dihydro-4-(3,4-dimethoxyphenyl)-2,6-dimethyl-1-[4-(4-methoxyphenyl)butyl]-3,5-pyridinedicarboxylate | 47 | Oil |

| Example No. | Starting material, $R^6$-X | Product | Yield (%) | M.P. (°C) |
|---|---|---|---|---|
| 9 | 3-Phenylpropyl bromide | Dimethyl 1,4-dihydro-4-(3,4-dichlorophenyl)-2,6-dimethyl-1-(3-phenylpropyl)-3,5-pyridinedicarboxylate | 52 | 98-99 |
| 10 | Benzyl bromide | Dimethyl 1,4-dihydro-4-(3,4-dimethoxyphenyl)-1-benzyl-3,5-pyridinedicarboxylate | 65 | 131-132 |
| 11 | 3-Phenylpropyl bromide | Dimethyl 1,4-dihydro-4-(4-chlorophenyl)-2,6-dimethyl-1-(3-phenylpropyl)-3,5-pyridinedicarboxylate | 55 | 126-128 |
| 12 | 3-Phenylpropyl bromide | Dimethyl 1,4-dihydro-4-(3,4,5-trimethoxyphenyl)-2,6-dimethyl-1-(3-phenylpropyl)-3,5-pyridinedicarboxylate | 45 | 130-131 |
| 13 | 3-Phenylpropyl bromide | Dimethyl 1,4-dihydro-4-(3-chlorophenyl)-2,6-dimethyl-1-(3-phenylpropyl)-3,5-pyridinedicarboxylate | 52 | 79-80 |
| 14 | 3-Phenylpropyl bromide | Dimethyl 1,4-dihydro-4-phenyl-2,6-dimethyl-1-(3-phenylpropyl)-3,5-pyridinedicarboxylate | 58 | 112-113 |
| 15 | 3-Phenylpropyl bromide | Dimethyl 1,4-dihydro-4-(4-fluorophenyl)-2,6-dimethyl-1-(3-phenylpropyl)-3,5-pyridinedicarboxylate | 55 | 89-90 |
| 16 | 3-Phenylpropyl bromide | Dimethyl 1,4-dihydro-4-(4-methoxyphenyl)-2,6-dimethyl-1-(3-phenylpropyl)-3,5-pyridinedicarboxylate | 56 | 116-117 |
| 17 | 3-Phenylpropyl bromide | Dimethyl 1,4-dihydro-4-(3-methoxyphenyl)-2,6-dimethyl-1-(3-phenylpropyl)-3,5-pyridinedicarboxylate | 56 | 104-105 |
| 18 | 3-Phenylpropyl bromide | Dimethyl 1,4-dihydro-4-(nitrophenyl)-2,6-dimethyl-1-(3-phenylpropyl)-3,5-pyridinedicarboxylate | 59 | 146-147 |

| Example No. | Starting material, $R^6$-X | Product | Yield (%) | M.P. (°C) |
| --- | --- | --- | --- | --- |
| 19 | 3-Phenylpropyl bromide | Dimethyl 1,4-dihydro-4-(3-nitrophenyl)-2,6-dimethyl-1-(3-phenylpropyl)-3,5-pyridinedicarboxylate | 54 | 123-124 |
| 20 | 3-Phenylpropyl bromide | Dimethyl 1,4-dihydro-4-(4-methylphenyl)-2,6-dimethyl-1-(3-phenylpropyl)-3,5-pyridinedicarboxylate | 59 | 117-118 |
| 21 | 3-Phenylpropyl bromide | Dimethyl 1,4-dihydro-4-(3-methylphenyl)-2,6-dimethyl-1-(3-phenylpropyl)-3,5-pyridinedicarboxylate | 57 | 93-94 |
| 22 | 3-Phenylpropyl bromide | Diethyl 1,4-dihydro-4-(3,4-dimethoxyphenyl)-2,6-dimethyl-1-(3-phenylpropyl)-3,5-pyridinedicarboxylate | 42 | oil |
| 23 | 3-Phenylpropyl bromide | Dibenzyl 1,4-dihydro-4-(3,4-dimethoxyphenyl)-2,6-dimethyl-1-(3-phenylpropyl)-3,5-pyridinedicarboxylate | 58 | oil |
| 24 | 3-Phenylpropyl bromide | Ethyl 1,4-dihydro-4-(3,4-dimethoxyphenyl)-1-(3-phenylpropyl)-3-cyano-5-pyridinecarboxylate | 79 | oil |
| 25 | 4-Cyclohexyl butyl bromide | Dimethyl 1,4-dihydro-4-(3,4-dimethoxyphenyl)-2,6-dimethyl-1-(4-cyclohexylbutyl) -3,5-pyridinedicarboxylate | 55 | oil |
| 26 | 3-Phenylpropyl bromide | Methyl 1,4-dihydro-4-(3,4-dimethoxyphenyl)-2,6-dimethyl-1-(3-phenylpropyl)-3-cyano-5-pyridinecarboxylate | 68 | oil |

| Example No. | Starting material, $R^6$-X | Product | Yield (%) | M.P. (°C) |
|---|---|---|---|---|
| 27 | 3-Phenylpropyl bromide | Dimethyl 1,4-dihydro-4-(3,4-dimethoxyphenyl)-2,6-diethyl-1-(3-phenylpropyl)-3,5-pyridinedicarboxylate | 35 | oil |
| 28 | 3-Phenylpropyl bromide | 1,4-Dihydro-4-(3,4-dimethoxyphenyl)-2,6-dimethyl-(3-phenylpropyl)-3,5-dicyanopyridine | 49 | oil |
| 29 | 3-Phenylpropyl bromide | Dimethyl 1,4-dihydro-4-(2-naphthyl -2,6-dimethyl-1-(3-phenylpropyl)-3,5-pyridinedicarboxylate | 28 | oil |
| 30 | 3-Phenylpropyl bromide | Dimethyl 1,4-dihydro-4-(1-naphthyl)-2,6-dimethyl-1-(3-phenylpropyl)-3,5-pyridinedicarboxylate | 42 | oil |
| 31 | 3-Phenylpropyl bromide | Dimethyl 1,4-dihydro-4-(3-methoxy,4-benzyloxyphenyl)-2,6-dimethyl-1-(3-phenylpropyl)-3,5-pyridinedicarboxylate | 47 | oil |
| 32 | 3-Phenylpropyl bromide | Dimethyl 1,4-dihydro-4-(3,4-diethoxy)-2,6-dimethyl-1-(3-phenylpropyl)-3,5 pyridinedicarboxylate | 53 | oil |
| 33 | 3-Phenylpropyl bromide | Ethyl 1,4-dihydro-4-(2-bromo,4,5-dimethoxyphenyl)-2,6-dimethyl-1-(3-phenylpropyl)-3-cyano-5-pyridinecarboxylate | 72 | oil |
| 34 | 3-Phenylpropyl bromide | Tert-butyl -1,4-dihydro-4-(3,4-dimethoxyphenyl)-2,6-dimethyl-1-(3-phenylpropyl)-3,5-pyridinedicarboxylate | 54 | oil |
| 35 | 3- Phenylpropyl bromide | Dimethyl 1,4-dihydro-4-(3,4-methylenedioxyphenyl)-2,6-dimethyl-1-(3-phenylpropyl)-3,5-pyridinedicarboxylate | 52 | 95-96 |
| 36 | 3-Phenylpropyl bromide | Dimethyl 1,4-dihydro-4-(2,3,4-trimethoxyphenyl)-2,6-dimethyl-1-(3-phenylpropyl)-3,5-pyridinedicarboxylate | 33 | oil |
| 37 | 3-Phenylpropyl bromide | Dimethyl 1,4-dihydro-4-(2,4,5-trimethoxyphenyl)-2,6-dimethyl-1-(3- phenyl propyl)-3,5-pyridinedicarboxylate | 60 | 157-158 |

| Example No. | Starting material, $R^6$-X | Product | Yield (%) | M.P. (°C) |
|---|---|---|---|---|
| 38 | 3-Phenylpropionyl chloride | Dimethyl 1,4-dihydro-4-(3,4-dimethoxyphenyl)-2,6-dimethyl-1-(3-phenylpropionyl) -3,5-pyridinedicarboxylate | 59 | oil |
| 39 | 3,3-Diphenylpropyl bromide | Dimethyl 1,4-dihydro-4-(3,4-dimethoxyphenyl)-2,6-dimethyl-1-(3,3-diphenylpropyl)-3,5-pyridinedicarboxylate | 67 | oil |
| 40 | Geranyl bromide | Dimethyl 1,4-dihydro-4-(3,4-dimethoxyphenyl)-2,6-dimethyl-1-geranyl-3,5-pyridinedicarboxylate | 25 | oil |
| 41 | 3-Phenylpropyl bromide | Dimethyl 1,4-dihydro-4-(2,4-dimethoxyphenyl)-2,6-dimethyl-1-(3-phenylpropyl)-3,5-pyridinedicarboxylate | 53 | 120-121 |
| 42 | 4-(3,4-Dimethoxyphenyl)-4-cyano-4-isopropyl bromide | Dimethyl 1,4-dihydro-4-(2,4-dimethoxyphenyl)-2,6-dimethyl-1-[4-(3,4-dimethoxyphenyl)-4-cyano-4-isopropylbutyl] -3,5-pyridinedicarboxylate | 35 | oil |

Example 43

Synthesis of 1,4-dihyadro-4-(3,4-dimethoxyphenyl)-2,6-dimethyl-1-(3-phenylpropyl)-3,5-pyridinecarboxylic acid-3-methylester

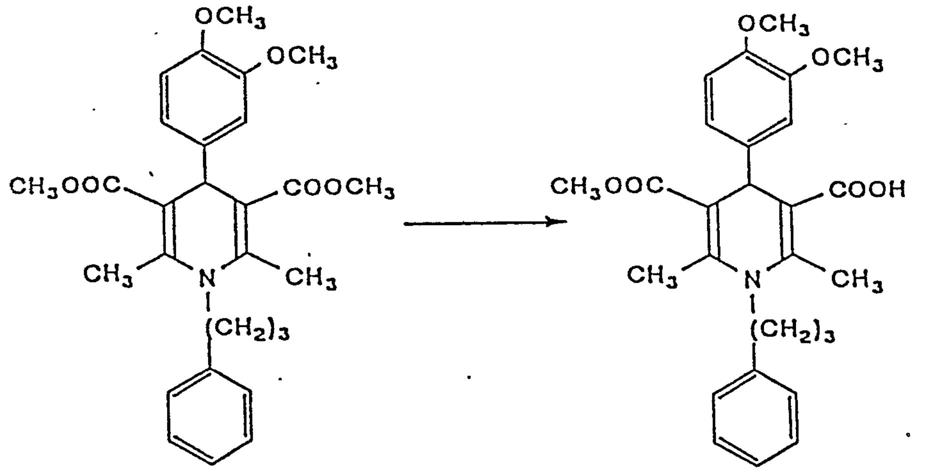

10 ml of 1N NaOH/water was added to a solution of 3.31g (6.9 mmol) of 1,4-dihydro-4-(3,4-dimethoxyphenyl)-2,6-dimethyl-1-(3-phenylpropyl)-3,5-pyridinecarboxylic acid-3,5-dimethyl ester in 15 ml of methanol. The mixture was cooled and extracted with $CH_2Cl_2$. The extract was dried and concentrated. The resulting residue was purified by column chromatography (SiO/AcEt: Hexane = 1:1) to give 0.75 g (2.1 mmol) of 1,4-dihydro-4-(3,4-dimethoxyphenyl)-2,6-dimethyl-2,6-dimethyl-1-(3-phenylpropyl)-3,5-pyridinecarboxylic acid-3-methyl ester.

Example 44

Synthesis of 1,4-dihydro-4-(3,4-dimethoxyphenyl)-2,6-dimethyl-1-(3-phenylpropyl)-3,5-pyridinedicarboxylic acid 3-monoethylester

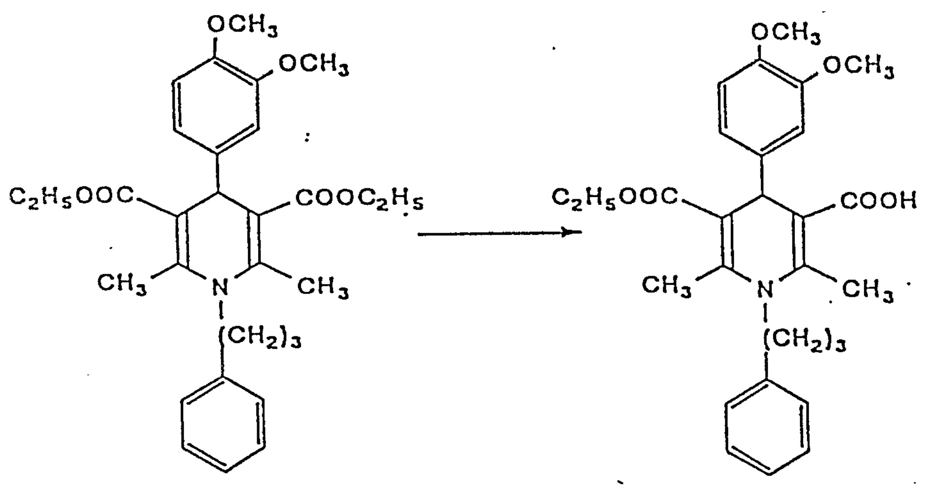

This synthesis was carried out in accordance with the method given for Example 47, except that column chromatography was carried out using 5% MeOH/$CH_2Cl_2$.

Example 45

Synthesis of 1,4-dihydro-4-(3,4-dimethoxyphenyl)-2,6-dimethyl-1-(3-phenylpropyl)-3-cyano-5-hydroxymethylpyridine

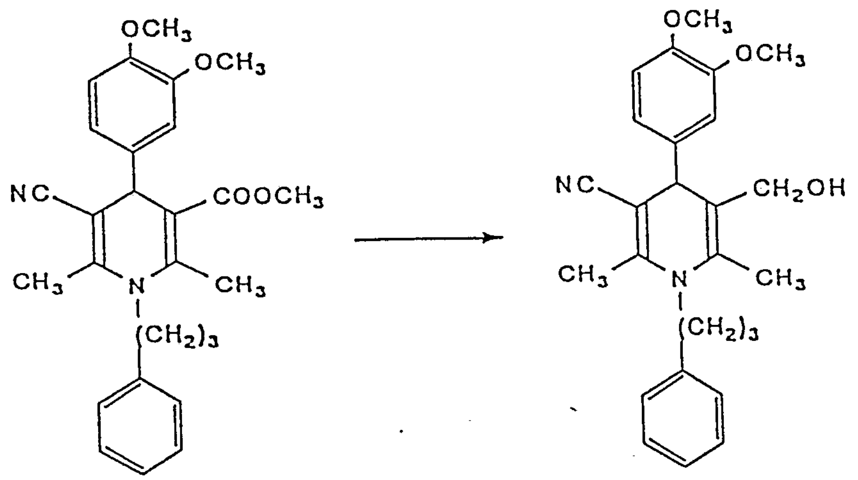

1.8 ml of 1N LiA1H4/Ether solution was added whilst stirring and at room temperature to a solution of 1.48 g (3.32 mmol) of 1.4-dihydro-4-(3,4-dimethoxyphenyl)-2,6-dimethyl-1-(3-phenylpropyl)-3-cyano-5-pyridine carboxylic acid-5-methylester in 42 ml of dry THF. The mixture was stirred for 20 minutes and was poured into ice-water. The mixture was filtered and extracted with $CH_2Cl_2$. This oil was purified by column chromatography (AcOEt/Hexane = 1:1) to give 1.08 g (2.58 mmol) of 1,4-dihydro-4-(3,4-dimethoxyphenyl)-2,6-dimethyl-1-(3-phenylpropyl)-3-cyano-5-hydroxymethyl-pyridine (78% oil).

Example 46

Synthesis of 1,4-dihydro-4-(3,4-dimethylphenyl)-2,6-dimethyl-1-(3-phenylpropyl)-3-cyano-5-formylpyridine

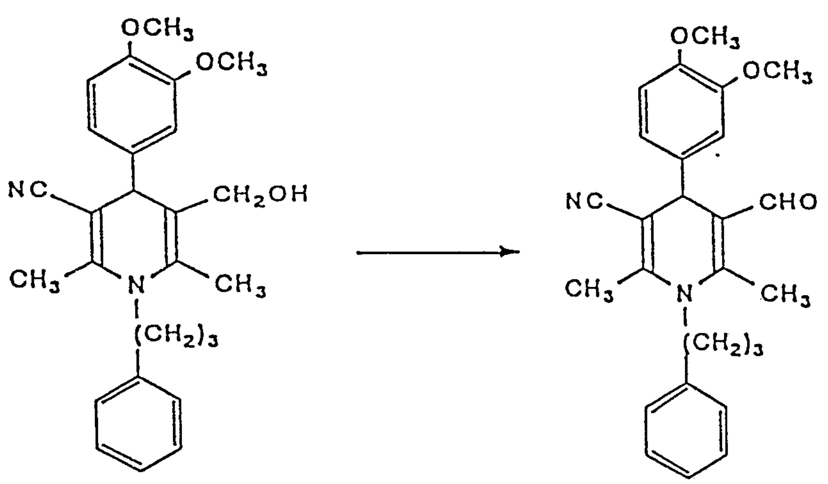

5 mg of activated manganese dioxide was added to a solution of 13 mg (0.026 mmol) of 1,4-dihydro-4-(3,4-dimethoxyphenyl)-2,6 dimethyl-1-(3-phenylpropyl)-3-cyano-5-hydroxy methylpyridine in 0.45 ml of dichloroethane. The mixture was heated at 80°C for 1 hour. The mixture was then filtered and concentrated. The resulting residue was purified by thin layer chromatography (AcOEt/Hexane = 1:1) to give 7.4 mg (0.015 mmol) of 1,4-dihydro-4-(3,4-dimethoxyphenyl)-2,6-dimethyl-1-(3-phenylpropyl)-3-cyano-5-formylpyridine (yield: 58%).

Example 47

Synthesis of 1,4-dihydro-4-(3,4-dimethoxyphenyl)-3,5-pyridinecarboxylic acid-3,5-dimethyl ester

A mixture of 4.3 g (51 mmol) methyl propiolate, 4.2g (26 mmol) of veratraldehyde, 4.5 g (58 mmol) of ammonium acetate and 4.5 ml of acetic acid were heated at 60°C for 20 min. The solution was poured into ice-water and the aqueous phase extracted with $CH_2Cl_2$. The extract was dried and concentrated to give a yellow oil. This oil was crystallized from methanol to give 2.8 g (8.4 mmol) of 1,4-dihydro-4-(3,4-dimethoxyphenyl)-3,5-pyridinecarboxylic acid-3,5-dimethyl ester (yield: 32%). This product was alkylated by using phenylpropyl bromide as described in Example 1 to give 2.5 g (5.5 mmol) of 1,4-dihydro-4-(3,4-dimethoxyphenyl)-1-(3-phenylpropyl)-3,5-pyridinecarboxylic acid-3,5-dimethylester (yield: 67%, m.p. 107-113°C).

Example 48 through 75

The compounds listed in the table below were prepared using procedures described in Example 1.

| Example No. | Product | |
|---|---|---|
| 48 | | R = 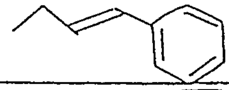 |
| 49 | | R = 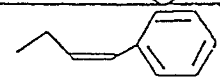 |
| 50 | | R = 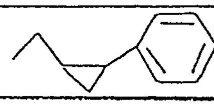 |
| 51 | | R = 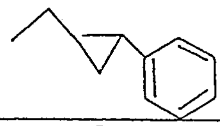 |
| 52 | 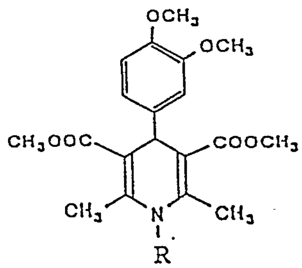 | R = 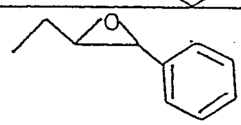 |
| 53 | | R= $-(CH_2)_5-CH_3$ |
| 54 | | R = $-CH_2CH_2CH_2CH_2CH=CH_2$ |
| 55 | | R = $-CH_2CH_2CH_2CH=CH_2$ |
| 56 | | R = 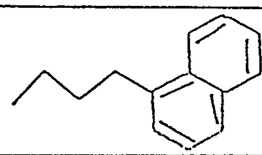 |
| 57 | | R = 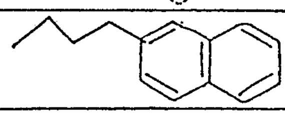 |
| 58 | | R = 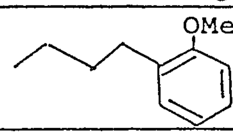 |
| 59 | | R = 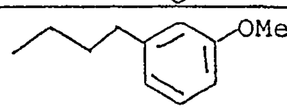 |
| 60 | | R = 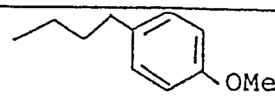 |
| 61 | | R = 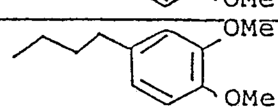 |
| 62 | | R = 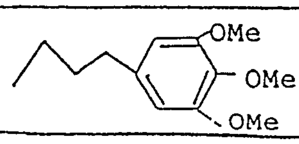 |

| Example No. | Product | |
|---|---|---|
| 63 | 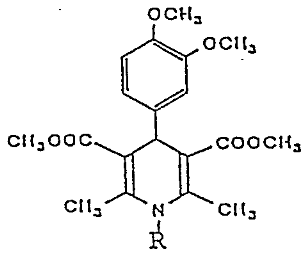 | R = 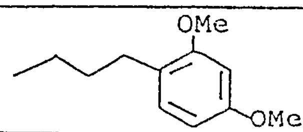 |
| 64 | | R = 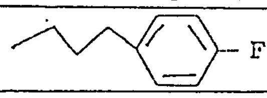 |
| 65 | | R = 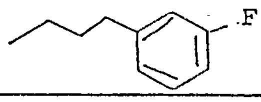 |
| 66 | | R = 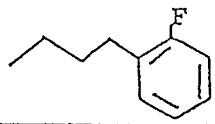 |
| 67 | | R = 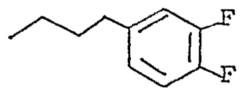 |
| 68 | | R = 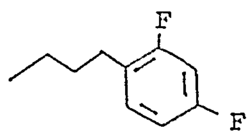 |
| 69 | | R = 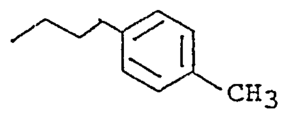 |
| 70 | | R = 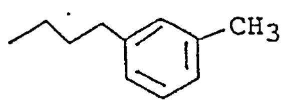 |
| 71 | | R = 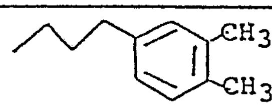 |
| 72 | | R = 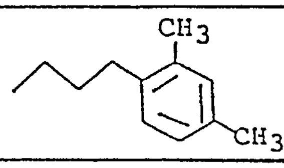 |
| 73 | 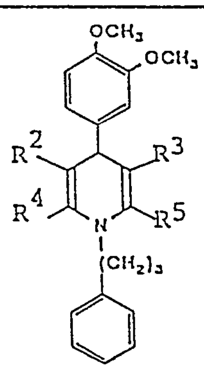 | $R^2$= -CHO $R^3$=-CHO<br>$R^4$= H $R^5$= H |
| 74 | | $R^2$= -CN $R^3$=-$CO_2CH_3$<br>$R^4$= -$CH_3$ $R^5$= -$CH_3$ (R-isomer) |
| 75 | | $R^2$= -CN $R^3$= -$CO_2CH_3$<br>$R^4$= -$CH_3$ $R^5$= - $CH_3$ (S-isomer) |

Example 76

Evaluation of activity of the 1,4-dihydropyridine derivatives on drug-resistant tumour cells

Vincristine (VCR) resistant tumour cells AD10, were dispensed to provide a concentration of 100 x $10^4$ cells/ml/well and incubated for one day. The medium was replaced with 0.5 ml of RPMI 1640 containing [$^3$H]VCR (10 x $10^4$ dpm/assay) 5%FBS, Kanamycin and 10mM Hepes B, and 5 µl of each test derivative solution (prepared by dissolving the derivative under test in DMSO, followed by dilution with PBS), were added to obtain final concentrations of 0.1 µg/ml and 1 µg/ml. After incubation in a $CO_2$-incubator for two hours, the cells were washed five times with 5 ml of ice-cooled PBS containing VCR. 0.5 ml of 0.2N NaOH solution was then added, and the mixture was transferred to vials, which were heated at 56° C for 30 to 60 minutes to lyse the cells. 24 mls of acid Aquasal was added and the amount of [$^3$H]VCR taken inside the cells was measured by means of a liquid scintillation counter. The effect of the test 1,4-dihydropyridine derivatives on the drug resistant tumour cells was evaluated by the percentage uptake of [$^3$H]VCR compared to percentage uptake of [$^3$H]VCR in a control (where there was no preincubation with a 1,4-dihydropyridine derivative). The results for some of the previous examples are tabulated below:

Effect Against Drug-Resistant Tumour Cells

| Example No. | % relative to control cells | |
|---|---|---|
| | Drug concn. 1.0 µg/ml | Drug concn. 10 µg/ml |
| 1 | 767 | 2058 |
| 2 | 122 | 157 |
| 3 | 144 | 458 |
| 4 | 461 | 1675 |
| 5 | 538 | 1769 |
| 6 | 386 | 1835 |
| 7 | 287 | 1573 |
| 8 | 268 | 1717 |
| 10 | 168 | 1462 |
| 12 | 356 | 1809 |
| 15 | 140 | 1075 |
| 16 | 151 | 1206 |
| 17 | 204 | 1284 |
| 18 | 160 | 943 |
| 24 | 278 | 1324 |
| 25 | 198 | 1114 |
| 26 | 356 | 2110 |
| 27 | 220 | 1048 |
| 28 | 348 | 1861 |
| 32 | 253 | 1625 |
| 45 | 223 | 1297 |
| 47 | 281 | 1835 |

Example 77

Evaluation of Side-Effects (hypotensive effect)

Six, well acclimatised, male SHR (spontaneous hypertensive rats) weighing 400 to 440 grams were used as test animals.

Each rat had a single intravenous injection of 1 ml physiological saline containing a test sample of the derivative (to provide a dose of 10 mg sample/kg body weight), 2.5% nicor and 2.5% ethanol. The blood pressure of the rats was measured by the plethysmographic tail method. The results obtained are tabulated below.

| Example No. | Time after administration (hr) | Maximum decrease in blood pressure (mmHg) |
|---|---|---|
| 2 | 0.5 | 0 |
| 3 | 0.5 | 0 |
| 4 | 0.5 | 0 |

From the foregoing, it can be seen that the 1,4-dihydropyridine derivatives of this invention are active on drug-resistant tumour cells and show low hypotensive effect. This invention is therefore of great value in the pharmaceutical industry.

The 1,4-dihydropyridine derivatives of the present invention are useful as agents against drug resistant tumour cells. The derivatives in combination with chemotherapeutic anti-cancer agents can be used to treat cancer by formulating them into preparations such as tablets, capsules, and elixirs for oral administration and into an aseptic liquid preparation or an aseptic suspension preparation for parenteral administration such as subcutaneous, intramuscular, intravenous injection, and suppositories. The 1,4-dihydropyridine derivatives in the present invention can be administered to a subject necessitating such treatment (animals and humans) in a dosage range of 1 to 1,000 mg per subject, generally several times a day, that is, in a total daily dosage of 1 to 3,000 mg. The dosage varies according to the seriousness of disease, the body weight of subjects, and other factors acknowledged by those skilled in the art.

To produce the preparations using the 1,4-dihydropyridine derivatives as described above for the present invention, they may be converted to dosage forms such as tablets, granules, powders, capsules, injections and suppositories by conventional methods.

For the production of oral preparations, there may be added to the 1,4-dihydropyridine derivative or to the mixture of 1,4-dihydropyridine derivative and chemotherapeutic anti-cancer agent, adjuvants such as fillers, binders, disintegrators, lubricants, colours, and correctives, as necessary, and then formed by conventional methods into tablets, coated tablets, granules, powders, capsules and the like.

Examples of specific materials which can be incorporated into tablets, capsules, and so forth are as follows: fillers such as cornstarch, lactose, white sugar, glucose, sorbitol, and crystalline cellulose; binders such as polyvinyl alcohol, polyvinyl ether, ethyl cellulose, methyl cellulose, gum arabic, tragacanth gelatine, shellac, hydroxypropyl cellulose, hydroxypropyl starch, polyvinyl pyrrolidone; disintegrators such as starch, agar, gelatine powder, crystalline cellulose, calcium carbonate, sodium bicarbonate, calcium citrate, dextrin and pectin; lubricants such as magnesium stearatem talc, polyethylene glycol, silica, hardened plant oil; colours such as one which is allowed as an additive for the medicines; correctives such as cocoa powder, mentha herb, aromatic acid, mentha oil, borneol, cinnamon bark powder. These tablets and granules may be coated with sugar, gelatine, or the like, as desired.

For the production of the injectable formulations, there may be added to the derivative or the mixture of 1,4-dihydropyridine derivative and chemotherapeutic anti-cancer agent, a pH adjusting agent, buffer agent, a stabilizing agent, preservatives or the like, as necessary to produce a material for subcutaneous, intramuscular or intravenous injection by conventional methods.

## Claims

1. A 1,4-dihydropyridine derivative of the formula (I)

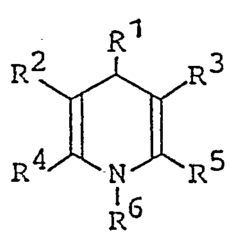

[I]

or a salt thereof for pharmaceutical use;
wherein:
$R^1$ is aryl which may optionally have one or more substituent groups;
$R^2$ and $R^3$ are the same or different selected from alkyl, alkyloxycarbonyl, aryloxycarbonyl, aralkyloxycarbonyl, aminocarbonyl, alkylaminocarbonyl, arylaminocarbonyl, hydroxycarbonyl, formyl,

hydroxyalkyl or cyano groups, all of which may optionally have one or more substituent groups;
$R^4$ and $R^5$ are the same or different selected from hydrogen, alkyl, hydroxymethyl, cyano, amino, formyl or halo groups;
$R^6$ is selected from alkyl, aralkyl, alkoxycarbonyl, or aralkyloxycarbonyl groups all of which may optionally have one or more substituent groups and wherein the alkyl segment may contain double or triple bonds or hetero atoms.

2. A 1,4-dihydropyridine derivative according to claim 1 wherein:
$R^1$ is phenyl or naphthyl;
$R^2$ and $R^3$ are the same or different selected from $C_{1-5}$ alkyl, alkyloxycarbonyl with $C_{1-5}$ alkyl segment, phenyloxycarbonyl, or phenylalkyloxycarbonyl; and $R^6$ is $C_{1-10}$ alkyl, phenylalkyl, alkyloxycarbonyl with $C_{1-5}$ alkyl segment, or phenylalkyloxycarbonyl.

3. A 1,4-dihydropyridine derivative according to claim 1 wherein $R_2$ and $R_3$ are the same or different selected from methyl, hydroxymethyl, methoxycarbonyl, ethoxycarbonyl, isopropyloxycarbonyl, methoxyethylcarbonyl, phenoxycarbonyl, benzyloxycarbonyl, aminocarbonyl, methylaminocarbonyl or benzylaminocarbonyl groups.

4. A 1,4-dihydropyridine derivative according to any preceding claim where $R^6$ is aralkyl, wherein the aralkyl group may optionally have a substituted or unsubstituted phenyl or phenoxy group at the end of its alkylene segment, and wherein the alkylene segment may optionally contain one or more substituent groups, selected from $C_{1-5}$ alkyl, nitrile or oxo groups and which may also contain one or more carbon-carbon double or triple bonds.

5. A 1,4-dihydropyridine derivative according to claim 1 wherein $R^1$ is a substituted aryl group and $R^6$ contains an aryl segment having one or more substituent groups selected from $C_{1-5}$ alkyl, $C_{1-5}$ alkyloxy, $C_{1-3}$ alkylene dioxy, halo, nitro, amino, nitrile or trifluoromethyl groups.

6. A 1,4-dihydropyridine derivative of the formula (II) or a salt thereof:

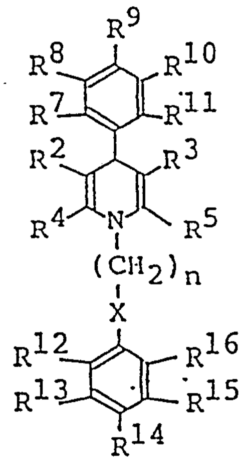

(II)

wherein:
$R^2$ and $R^3$ are the same or different selected from methyl, hydroxymethyl, tert-butoxycarbonyl, methoxycarbonyl, ethoxycarbonyl, isopropyloxycarbonyl, methoxyethyloxycarbonyl, phenoxycarbonyl, benzyloxycarbonyl, aminocarbonyl, methylaminocarbonyl, benzylaminocarbonyl, hydroxycarbonyl, formyl and cyano groups;
$R^4$ and $R^5$ are the same or different selected from hydrogen, methyl or ethyl;
$R^7$, $R^8$, $R^9$, $R^{10}$ and $R^{11}$ are the same or different selected from hydrogen, halogen, $C_{1-5}$ alkyl, hydroxy, $C_{1-5}$ alkyloxy, benzyloxy, nitro, amino, nitrile or trifluoromethyl, and wherein any neighbouring two of $R^7$, $R^8$, $R^9$, $R^{10}$ and $R^{11}$ may be bonded by a $C_{1-3}$ alkylene dioxy group;
$R^{12}$, $R^{13}$, $R^{14}$, $R^{15}$ and $R^{16}$ are the same or different selected from hydrogen, halogen, C1-5 alkyl, hydroxy, $C_{1-5}$ alkyloxy, nitro, amino, nitrile, benzyloxy or trifluoromethyl; and wherein any neighbouring two of $R^{12}$, $R^{13}$, $R^{14}$, $R^{15}$ and $R^{16}$ may be bonded by a $C_{1-3}$ alkylene dioxy group;
n is an integer of 2 to 9;
X is methylene, carbonyl, oxygen or nitrogen, wherein where X is methylene it may be optionally substituted with a group selected from $C_{1-5}$ alkyl, nitrile.

7. A 1,4-dihydropyridine derivative selected from
dimethyl 1,4-dihydro-4-(3,4-dimethoxyphenyl)-2,6-dimethyl-1-(3-phenylpropyl)-3,5-pyridinedicarboxylate, dimethyl 1,4-dihydro-4-(3,4-dimethoxyphenyl)-2,6-dimethyl-1-(3-phenoxypropyl)- 3,5-pyridinedicarboxylate, dimethyl 1,4-dihydro-4-(3,4-dimethoxyphenyl)-2,6-dimethyl-1-(4-phenylbutyl)-3,5-pyridinedicarboxylate, dimethyl 1,4-dihydro-4-(3,4-dimethoxyphenyl)-2,6-dimethyl-1-(5-phenylpentyl)-3,5-pyridinedicarboxylate, dimethyl 1,4-dihydro-4-(3,4-dimethoxyphenyl)-2,6-dimethyl-1-(4-phenoxybutyl)-3,5-pyridinedicarboxylate, dimethyl 1,4-dihydro-4-(3,4-dimethoxyphenyl)-2,6-dimethyl-1-[4-(4-methoxyphenyl)butyl]-3,5-pyridinedicarboxylate, dimethyl 1,4-dihydro-4-(3,4-dichlorophenyl)-2,6-dimethyl-1-(3-phenylpropyl)-3,5-pyridinedicarboxylate, dimethyl 1,4-dihydro-4-(4-chlorophenyl)-2,6-dimethyl-1-(3-phenylpropyl)-3,5-pyridinedicarboxylate, dimethyl 1,4-dihydro-4-(3,4,5-trimethoxyphenyl)-2,6-dimethyl-1-(3-phenylpropyl)-3,5-pyridinedicarboxylate, dimethyl 1,4-dihydro-4-(3-chlorophenyl)-2,6-dimethyl-1-(3-phenylpropyl)-3,5-pyridinedicarboxylate, dimethyl 1,4-dihydro-4-phenyl-2,6-dimethyl-1-(3-phenylpropyl)-3,5-pyridinedicarboxylate, dimethyl 1,4-dihydro-4-(4-fluorophenyl)-2,6-dimethyl-1-(3-phenylpropyl)-3,5-pyridinedicarboxylate, dimethyl 1,4-dihydro-4-(4-methoxyphenyl)-2,6-dimethyl-1-(3-phenylpropyl)-3,5-pyridinedicarboxylate, dimethyl 1,4-dihydro-4-(3-methoxyphenyl)-2,6-dimethyl-1-(3-phenylpropyl)-3,5-pyridinedicarboxylate, dimethyl 1,4-dihydro-4-(nitrophenyl)-2,6-dimethyl-1-(3-phenylpropyl)-3,5-pyridinedicarboxylate, dimethyl 1,4-dihydro-4-(3-nitrophenyl)-2,6-dimethyl-1-(3-phenylpropyl)-3,5-pyridinedicarboxylate, dimethyl 1,4-dihydro-4-(4-methylphenyl)-2,6-dimethyl-1-(3-phenylpropyl)-3,5-pyridinedicarboxylate, dimethyl 1,4-dihydro-4-(3-methylphenyl)-2,6-dimethyl-1(3-phenylpropyl)-3,5-pyridinedicarboxylate, dimethyl 1,4-dihydro-4-(3,4-dimethoxyphenyl)-2,6-dimethyl-1-(3-phenylpropyl)-3,5-pyridinedicarboxylate, dibenzyl 1,4-dihydro-4-(3,4-dimethoxyphenyl)-2,6-dimethyl-1-(3-phenylpropyl)-3,5-pyridinedicarboxylate, ethyl 1,4-dihydro-4-(3,4-dimethoxyphenyl)-1-(3-phenylpropyl)-3-cyano-5-pyridinedicarboxylate, dimethyl , methyl 1,4-dihydro-4-(3,4-dimethoxyphenyl)-2,6-dimethyl-1-(3-phenylpropyl)-3-cyano-5-pyridinedicarboxylate, dimethyl 1,4-dihydro-4-(3,4-dimethoxyphenyl)-2,6-dimethyl-1-(3-phenylpropyl)-3,5-pyridinedicarboxylate, 1,4-dihydro-4-(3,4-dimethoxyphenyl)-2,6-dimethyl-1-(3-phenylpropyl)-3,5-dicyanopyridine, dimethyl 1,4-dihydro-4-(2-naphthyl)-2,6-dimethyl-1-(3-phenylpropyl)-3,5-pyridinedicarboxylate, dimethyl 1,4-dihydro-4-(1-naphthyl)-2,6-dimethyl--1-(3-phenylpropyl)-3,5-pyridinedicarboxylate, dimethyl 1,4-dihydro-4-(3-methoxy, 4-benzyloxyphenyl)-2,6- dimethyl-1-(3-phenylpropyl)-3,5-pyridinedicarboxylate, dimethyl 1,4-dihydro-4-(3,4-dimethoxy)-2,6-dimethyl-1-(3-phenylpropyl)-3,5-pyridinedicarboxylate, ethyl 1,4-dihydro-4-(2-bromo,4,5-dimethoxyphenyl)-2,6-dimethyl-1-(3-phenylpropyl)-3-cyano-5-pyridinedicarboxylate, tert-butyl-1,4-dihydro-4-(3,4-dimethoxyphenyl)-2,6-dimethyl-1-(3-phenylpropyl)-3,5-pyridinedicarboxylate, dimethyl 1,4-dihydro-4-(3,4-methylenedioxyphenyl)-2,6-dimethyl-1-(3-phenylpropyl)-3,5-pyridinedicarboxylate, dimethyl 1,4-dihydro-4-(2,3,4-trimethoxyphenyl)-2,6-dimethyl-1-(3-phenylpropyl)-3,5-pyridinedicarboxylate, dimethyl 1,4-dihydro-4-(2,4,5-trimethoxyphenyl)-2,6-dimethyl-1-(3-phenylpropyl)-3,5-pyridinedicarboxylate, dimethyl 1,4-dihydro-4-(3,4-dimethoxyphenyl)-2,6-dimethyl-1-(3-phenylpropionyl)-3,5-pyridinedicarboxylate, dimethyl 1,4-dihydro-4-(3,4-dimethoxyphenyl)-2,6-dimethyl-1-(3,3-diphenylpropyl)-3,5-pyridinedicarboxylate, dimethyl 1,4-dihydro-4-(2,4-dimethoxyphenyl)-2,6-dimethyl-1-(3-phenylpropyl)-3,5-pyridinedicarboxylate, dimethyl 1,4-dihydro-4-(2,4-dimethoxyphenyl)-2,6-dimethyl-1-[4-(3,4-dimethoxyphenyl)-4-cyano-4-isopropylbutyl]-3,5-pyridinedicarboxylate, 1,4-dihydro-4-(3,4-dimethoxyphenyl)-2,6-dimethyl-1-(3-phenylpropyl)- 3,5-pyridinedicarboxylic acid-3-methylester, 1,4-dihydro-4-(3,4-dimethoxyphenyl)-2,6-dimethyl-1-(3-phenylpropyl)-3,5-pyridinedicarboxylic acid 3-monoethylester, 1,4-dihydro--4-(3,4-dimethoxyphenyl)-2,6-dimethyl-1-(3-phenylpropyl)-3-cyano-5-hydroxymethylpyridine, 1,4-dihydro-4-(3,4-dimethylphenyl)-2,6-dimethyl-1-(3-phenylpropyl)-3-cyano-5-formylpyridine, 1,4-dihydro-4-(3,4-dimethoxyphenyl)-3,5-pyridinecarboxylic acid-3,5-dimethyl ester.

8. A 1,4-dihydropyridine derivative of the formula:

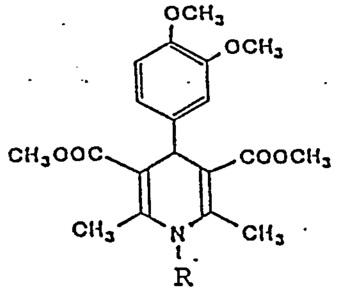

wherein R is selected from

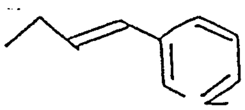
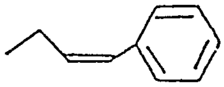
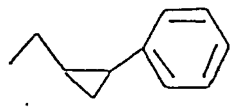

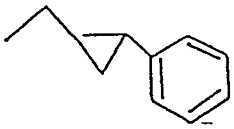
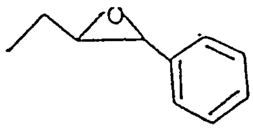

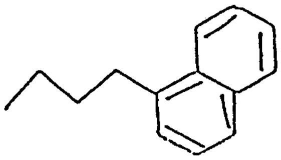
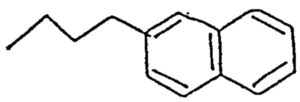
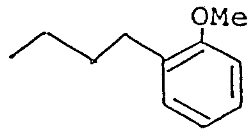

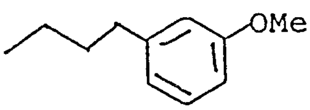
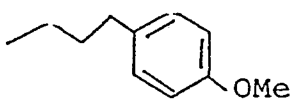
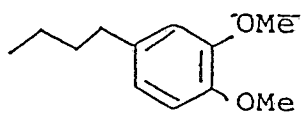

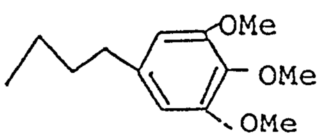
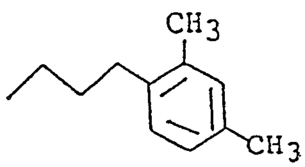
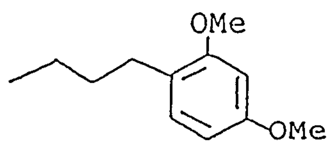

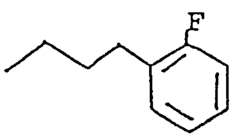
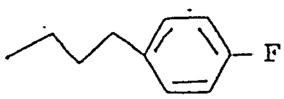
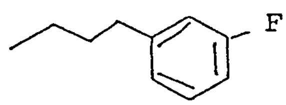

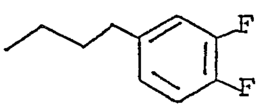
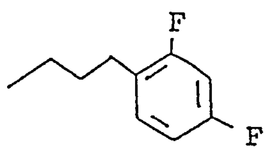
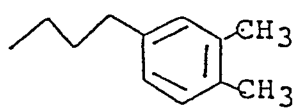

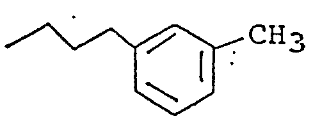
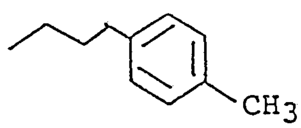

9. A 1,4-dihydropyridine derivative of the formula:

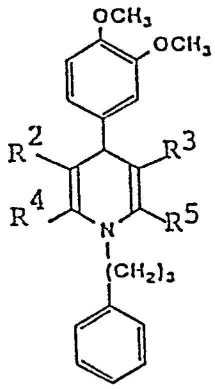

wherein (i) $R^2$ and $R^3$ are CHO and $R^4$ and $R^5$ are H; or (ii) $R^2$ is CN, $R^3$ is $CO_2CH_3$, $R^4$ and $R^5$ are $CH_3$ and the derivatives being
(i) 1,4 dihydro-4-(3,4-dimethoxyphenyl)-3,4-diformyl-1-(3-phenylpropyl) pyridine; and
(ii) 1,4-dihydro-4-(3,4-dimethoxyphenyl)-2,6-dimethyl-5-cyano-4-carboxylic acid 4-methylester
wherein (ii) may be an R or S isomer or a mixture thereof.

10. A 1,4-dihydropyridine derivative or a salt thereof for pharmaceutical use selected from dimethyl 1,4-dihydro-4-(3,4-dimethoxyphenyl)-2,6-dimethyl-1-methyl-3,5-pyridinedicarboxylate, dimethyl 1,4-dihydro-4-(3,4-dimethoxyphenyl)-2,6-dimethyl-1-ethoxycarbonyl-3,5-pyridinedicarboxylate, dimethyl 1,4-dihydro-4-(3,4-dimethoxyphenyl)-1-benzyl-3,5-pyridinedicarboxylate, dimethyl 1,4-dihydro-4-(3,4-dimethoxyphenyl)-2,6-dimethyl-1-(4-cyclohexylbutyl)-3,5-pyridinecarboxylate, dimethyl 1,4-dihydro-4-(3,4-dimethoxyphenyl)-2,6-dimethyl-1-geranyl-3,5-pyridinedicarboxylate,

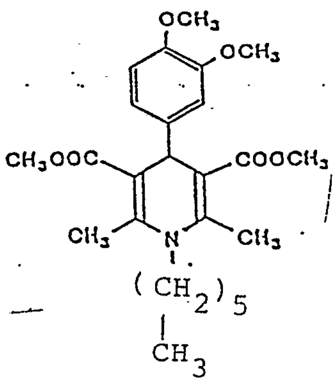

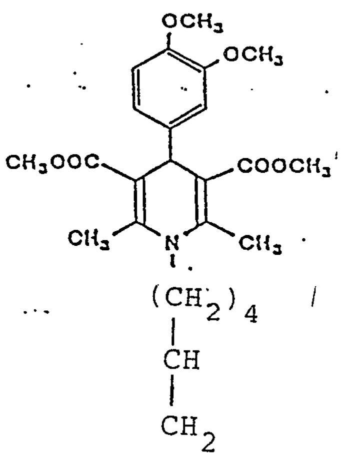

or

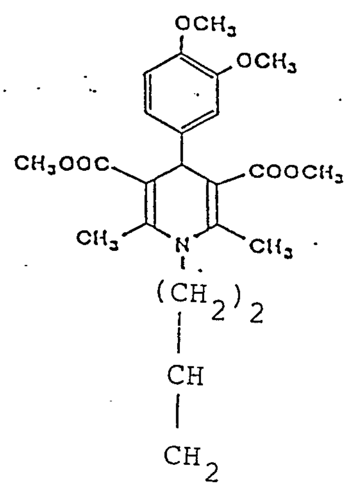

11. A compound of any one of claims 6 to 9 for pharmaceutical use.

12. The use of a compound of any one of claims 1 to 10 in the preparation of a medicament for use in treatment of tumour cells.

13. A pharmaceutical composition comprising a compound of any one of claims 1 to 10 and a suitable excipient.

14. Product containing a compound of any one of claims 1 to 10 and a chemotherapeutic anti-cancer agent as a combined preparation for simultaneous, separate or sequential use in the treatment of tumour cells.